# EUROPEAN PATENT APPLICATION

(11) **EP 3 095 851 A1**
(43) Date of publication of application: **23.11.2016**
(21) Application number: 14878562.9
(22) Date of filing: 07.11.2014
(51) Int. Cl.: C12M 1/34, G01N 1/02

(54) **METHOD FOR PRODUCING DETECTION SUBJECT COLLECTION TOOL**

(30) Priority: 17.01.2014 JP 2014006703
(71) Applicant: Hitachi Plant Services Co., Ltd., Tokyo 170-6034 (JP)
(72) Inventor: NAKATSUKA Yuta, Tokyo 100-8280 (JP); MIYASHITA Noe, Tokyo 100-8280 (JP); MORI Shuichi, Tokyo 100-8280 (JP); SEO Koji, Tokyo 100-8280 (JP)
(74) Representative: Beetz & Partner mbB
(86) International application number: PCT/JP2014/079542
(87) International publication number: WO 2015/107756

(57) **Abstract**

Provided is a method for producing an instrument of collecting detection target. The instrument includes a filter unit and a carrier unit that holds a carrier of collecting a detection target. The method comprises at least: a tool ATP eliminating step of eliminating ATP from tools to be used in the production steps, a mixture ATP eliminating step of eliminating ATP from a carrier prepared by mixing raw materials via using the tools from which ATP is thus eliminated, and a filling step of filling the carrier from which ATP is thus eliminated into a carrier unit.

## Description

### Technical Field

The present invention relates to a method for producing an instrument of collecting detection target. Specifically, the instrument (or called target collecting instrument) is used for a method for detecting a biomaterial with high measurement sensitivity such as an ATP method or the like.

### Background Art

Recently, for counting microorganisms in a short time and with high sensitivity, there have been proposed methods of specifically labeling a component of microorganism cells using a luminescent reagent or a fluorescent reagent to measure luminescence intensity. For example, in an ATP method, a chemical substance such as ATP (Adenosine Triphosphate) inevitably contained in a viable cell is measured as an indicator of calculating the number of microorganisms. This measurement may be completed within several minutes to several tens of minutes.

In the ATP method, measured is bioluminescence generated by a luminescence reaction between luciferase: an enzyme derived from a viable microorganism and luciferin: a substrate protein of the luciferase (e.g., see Patent Document 1). Herein, ATP is extracted from a sample including microorganisms, and the extracted ATP is made to be luminescent by added with a mixed solution of luciferin and luciferase. Hence, the ATP amount is calluculated by the luminescence intensity (e.g., see Patent Document 2).

Further, as a method for counting microorganisms via the ATP method, Patent Document 3 discloses a method for collecting and detecting microorganisms floating in the air, easily and in a short time. In that method, provided is a carrier that is in a gel state at the time of collecting microorganisms but transformed into a sol state at the time of detection, allowing a reaction of a detection reagent in a sol carrier.

Moreover, Patent Document 4 also discloses a method for collecting and detecting microorganisms floating in the air. Specifically, disclosed is a technique for automatically collecting and detecting microorganisms by using an instrument of collecting detection object. The instrument includes a collection dish holding a carrier arranged at one side of the instrument to collect a detection object, and a discharge outlet arranged at the other side of the instrument to have a filter of separating the detection object.

However, when an extremely small amount of ATP is targeted for the measurement in the above detecting methods, the resulting luminescent amount falls in extremely small one. This leads to a drawback caused by influence of residual ATP remained in the measurement system and contamination of non-targeted ATP in the measurement, which results in failure to achieve high measurement accuracy.

On the contrary, Patent Documents 5 and 6 disclose a luminescent measuring apparatus capable of eliminating residual ATP adhering to a nozzle for dispensing a reagent, by cleaning the nozzle with a luminescent reagent solution and a cell lysate solution to suppress background luminescence derived from the residual ATP. Hereby, the apparatus accomplishes the luminescent measurement in a short time and with high sensitivity. However, Patent Documents 5 and 6 focus on only the residual ATP adhering to a nozzle without taking any care of other adhering ATP.

### Citation List

### Patent Documents

Patent Document 1: Japanese Patent Publication No.2070780
Patent Document 2: Japanese Unexamined Patent Application Publication No.H11-155597
Patent Document 3: Japanese Unexamined Patent Application Publication No.2012-205600
Patent Document 4: Japanese Unexamined Patent Application Publication No.2011-133444
Patent Document 5: Japanese Unexamined Patent Application Publication No.2008-249628
Patent Document 6: Japanese Unexamined Patent Application Publication No.2011-149721

### Summary of Invention

### Technical Problem

As mentioned hereinbefore, in conventional techniques, there has been known an apparatus for washing residual ATP adhering to a nozzle for dispensing a reagent. However, in such a conventional apparatus, little care is taken of ATP contained in a carrier of collecting a measurement object (i.e., detection target) thus existed before the collection as well as residual ATP adhering to a measurement object collecting instrument (i.e., instrument of collecting measurement object) also thus existed before the collection. Accordingly, this residual ATP causes a drawback that high measurement accuracy cannot be achieved when such a conventional instrument is used for measuring ATP present in an extremely small amount.

In view of the above drawbacks in the conventional techniques, an object of the present invention is to provide a method for producing an instrument of collecting detection target. This producing method enables the instrument containing no residual ATP to be obtained due to the step of eliminating residual ATP adhering to or contained in the instrument of collecting detection target.

### Means for Solving Problem

For solving the above drawbacks, the present invention is directed to a method for producing an instrument of collecting detection target, the instrument including a carrier unit that holds a carrier of collecting a detection target. This producing method includes a tool ATP eliminating step of eliminating ATP adhering to tools to be used in production steps, and a carrier ATP eliminating step of eliminating ATP adhering to a carrier made via mixing raw materials by the tools from which ATP is eliminated at the tool ATP eliminating step.

Further, the producing method additionally includes a filling step of filling the carrier from which ATP is eliminated in the carrier ATP eliminating step into the carrier unit.

Further, the producing method additionally includes a molding step of molding the carrier thus filled in the carrier unit in the previous filling step.

Moreover, in the producing method, the tool ATP eliminating step may be a step of eliminating ATP by immersing the tools in a liquid ATP eliminating reagent, or a step of eliminating ATP by spraying a misty ATP eliminating reagent onto the tools.

Furthermore, in the producing method, the carrier ATP eliminating step may be a step of eliminating ATP by adding an ATP eliminating reagent to a carrier made via mixing raw materials.

In the producing method, the ATP eliminating reagent includes at least one selected from a group of apyrase, alkaline phosphatase, acidic phosphatase, hexokinase, adenosine triphosphatase, and adenosine phosphate deaminase.

Further, the producing method additionally includes a step of sterilizing tools to be used prior to the tool ATP eliminating step in the production steps, a step of packing the target collecting instrument thus treated in the molding step, and a step of sterilizing the target collecting instrument thus packed in the previous step. Herein, the steps from the tool ATP eliminating step to the packing step are carried out in a sterile operation area.

### Advantageous Effect

According to the producing method of the present invention, an instrument of collecting detection target, the instrument containing no residual ATP, may be produced. Further, use of the instrument of collecting detection target enables an extremely small amount of ATP to be measured with high accuracy.

### Brief Description of Drawings

FIGS. 1A and 1B are exploded perspective views each showing a carrier unit 1 included in an instrument of collecting detection target in an Example of the present invention. FIG. 1A is an exploded perspective view of the instrument when looked down relative to an oblique upper side. FIG. 1B is an exploded perspective view when looked up relative to an oblique lower side.
FIGS. 2A and 2B are exploded perspective views each showing a filter unit 11 included in the instrument of collecting detection target in an Example of the present invention. FIG. 2A is an exploded perspective view of the instrument when looked down relative to an oblique upper side. FIG. 2B is an exploded perspective view when looked up relative to an oblique lower side.
FIG. 3 is an exploded view of an air sampler attached to the instrument of collecting detection target (e.g., carrier unit) in an Example of the present invention.
FIG. 4 is a perspective view showing the instrument of collecting detection target in an Example of the present invention, and surroundings of a mounting part of a microorganism counter.
FIG. 5 is a flow chart showing production steps of the instrument of collecting detection target in an Example of the present invention.
FIG. 6 is a schematic diagram explaining the production steps of the instrument of collecting detection target in Example 1 of the present invention.
FIG. 7 is a schematic diagram explaining the production steps of the instrument of collecting detection target in Example 2 of the present invention.

### Embodiments for Carrying Out Present Invention

Hereinafter, embodiments for carrying out the present invention will be described in detail appropriately referring to attached drawings. First, an instrument of collecting detection target will be explained, which is configured to collect a detection target such as microorganisms. The instrument of collecting detection target includes a carrier unit 1 and a filter unit 11.

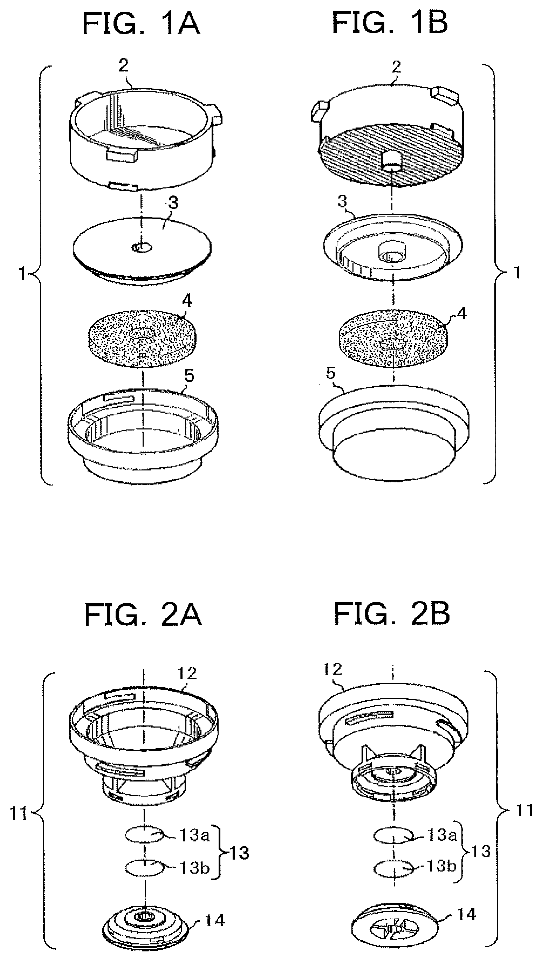

FIGS. 1A and 1B are exploded perspective views each showing a carrier unit 1 included in an instrument of collecting detection target in an Example of the present invention. FIG. 1A is an exploded perspective view of the instrument when looked down relative to an oblique upper side. FIG. 1B is an exploded perspective view when looked up relative to an oblique lower side. The carrier unit 1 includes a lid 2, a collection dish 3, a carrier 4, and a carrier cover 5. The carrier 4 is a gel material for sticking and collecting a detection target, and prepared as a mixture by mixing raw materials of the carrier 4. The collection dish 3 is configured to hold the carrier 4. The lid 2 is configured to attach the collection dish 3 holding the carrier 4 to an air sampler 40. The carrier cover 5 is a cover for preventing contamination of the carrier 4 by covering the carrier 4 held in the collection dish 3.

FIGS. 2A and 2B are exploded perspective views each showing a filter unit 11 included in the instrument of collecting detection target in an Example of the present invention. FIG. 2A is an exploded perspective view of the instrument when looked down relative to an oblique upper side. FIG. 2B is an exploded perspective view when looked up relative to an oblique lower side. The filter unit 11 includes a housing 12, a filter 13, and a filter fixing ring 14.

After the detection target has been collected, the housing 12 is connected with the carrier unit 1 (i.e., in the state of detaching the carrier cover 5), thereby to protect the carrier 4 so that the detection target thus separated by the filter 13 reacts with a test reagent in the housing 12. The filter 13 is configured to separate the detection target. Herein, the filter 13 has a double structure including a hydrophilic filter 13a and a hydrophobic filter 13b. A liquid can be held in an upper portion of the filter due to the property of the hydrophobic filter 13b so long as suction or pressure filtration is not performed. The filter fixing ring 14 is configured to fix the filter 13 onto the housing 12.

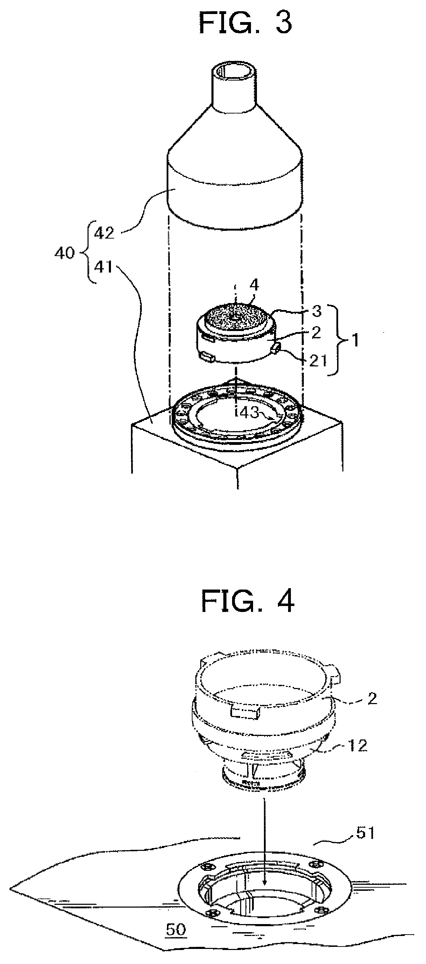

When microorganisms (i.e., detection target) are collected, an air sampler (i.e., collection apparatus) 40 is used for the collection. FIG. 3 is an exploded view of an air sampler 40 attached to the instrument of collecting detection target (e.g., carrier unit 1) in an Example of the present invention. The air sampler 40 includes an air sampler body 41 and an air sampler lid 42. The carrier unit 1 is attached to the air sampler body 41 in the state of detaching the carrier cover 5. At that time, a connector 21 included in the lid 2 of the carrier unit 1 is connected with a connector receiver 43 included in the air sampler body 41.

The air sampler body 41 attaching the carrier unit 1 is combined with the air sampler lid 42 to receive an air flow generated when air is sucked, so that the carrier 4 collects microorganisms suspended in the air. After completion of the collection, the carrier unit 1 is turned upside down to locate the carrier 4 at the down side so that the lid 2 is connected with the housing 12 of the filter unit 11.

FIG. 4 is a perspective view showing the instrument of collecting detection target (or called target collecting instrument) in an Example of the present invention, and surroundings of a mounting part 51 of a microorganism counter 50. The target collecting instrument thus having completed the collection step is mounted on the mounting part 51 of the microorganism counter 50 to count the microorganisms. When the target collecting instrument is to be mounted, as shown in FIG. 4, the target collecting instrument is turned upside down as reversed in FIG. 3 so that the lid 2 locates at the upper side. After the target collecting instrument is mounted on the microorganism counter 50, only the lid 2 is detached and the microorganism counter 50 automatically performs the counting measurement (See the measurement in Patent Document 4).

Hereinafter, a method for producing the instrument of collecting detection target (i.e., microorganisms) as described hereinbefore (or called target collecting instrument) will be explained specifically based on the following Examples. The carrier unit 1 and the filter unit 11 included in the target collecting instrument are independently prepared and packed in the production step. Then, the packages of the carrier unit 1 and the filter unit 11 are independently opened at a collection site and a measurement site of microorganisms, to use both the units 1 and 11.

### Examples

### (Example 1)

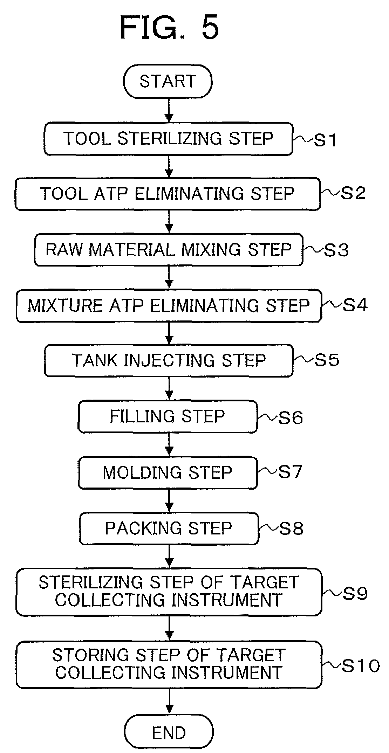

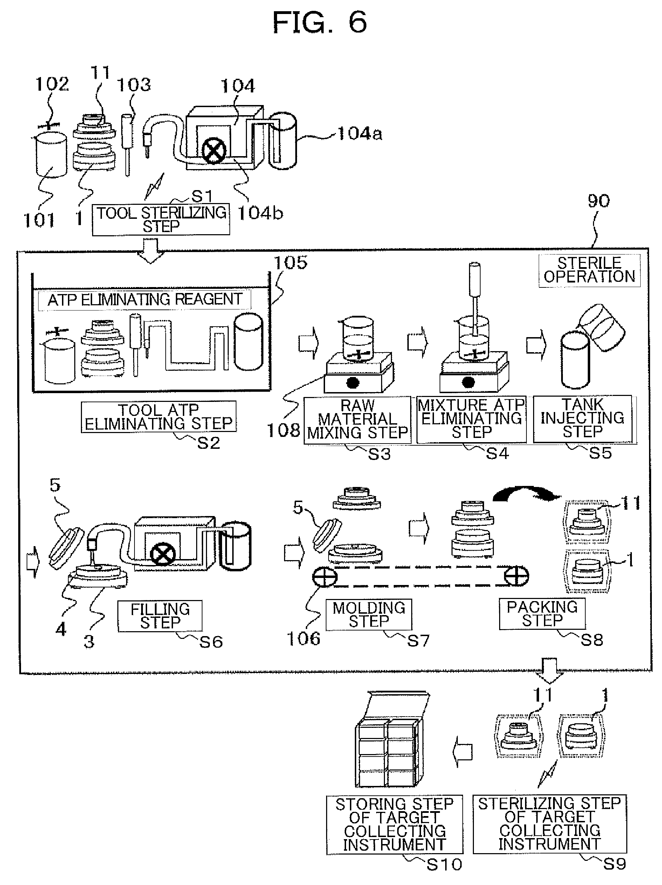

Example 1 of the present invention will be explained referring to FIGS. 5 and 6. First, tools to be used in the production steps are prepared. The tools to be used in the production steps include a beaker 101, a stirring bar 102 for mixing raw materials, a pipette 103 for adding a reagent, a carrier unit 1 excluding a carrier 4 included in the instrument of collecting detection target, a filter unit 11, a tubing pump 104 for filling the carrier 4, and a tank 104a and a tube 104b both of which are components of the tubing pump 104.

Among the tools to be used in the production steps, the filter unit 11 and the carrier unit 1 including the carrier 4 are packed and delivered as products, while the remaining tools are used only in the production steps.

At the first step, each of the above described tools to be used in the production steps is sterilized. The sterilization is performed by EOG sterilization (S1: tool sterilizing step in FIGS. 5 and 6). The EOG sterilization is performed by ethylene oxide gas under low temperature and low humidity conditions. Note, besides the EOG sterilization, examples of sterilization methods include gamma ray sterilization, electron beam sterilization, steam sterilization, dry heat sterilization, and flaming sterilization or the like. In the tool sterilizing step S1, microorganisms having ATP are sterilized, thereby to eliminate a source of ATP generation.

Next, each of the above tools is brought to a clean bench 90 in a clean room serving as a sterile operation area with keeping the sterile condition made by the sterilizing step as described above. The steps to be operated in the clean bench 90 thereafter are performed via automatic operation or manual operation.

Then, an ATP eliminating reagent at a predetermined concentration (e.g., attached to Lucifell HS Set (60315)™ / Kikkoman) is added to a solution in an immersion container 105. Each of the tools is immersed in the immersion container 105 containing the liquid ATP eliminating reagent to eliminate ATP from a surface of each tool possibly contacting with the carrier 4 and the raw materials thereof (S2: tool ATP eliminating step in FIGS. 5 and 6). In the tool ATP eliminating step S2, residual ATP independently of microorganisms can be eliminated.

The ATP eliminating reagent includes at least one selected from a group of apyrase, alkaline phosphatase, acidic phosphatase, hexokinase, adenosine triphosphatase, and adenosine phosphate deaminase.

Then, the raw materials of the carrier 4 and the stirring bar102 are put in the beaker 101, and the raw materials of the carrier 4 are mixed and stirred in the beaker 101 with the stirring bar 102 by the stirrer 108 (S3: raw material mixing step in FIGS. 5 and 6). The raw materials of the carrier 4 are brought to the clean bench 90 in advance after subjected to a sterilizing step different from the tool sterilizing step S1).

The carrier 4 has a property that it is in a gel state at the collection timing but is transformed into a sol state at the measurement timing of microorganisms (i.e., performing a sol-gel transformation). When gelatin, glycerol, distilled water are used as the raw materials, a carrier that temperature-dependently performs a sol-gel transformation may be prepared. Note, in the present Example, gelatin, glycerol, distilled water are used as the raw materials. However the present invention is not limited to those materials. For example, a mixture of variety materials, being in a gel state at the collection timing but transformed into a sol state at the measurement timing, may be used as the raw materials of the carrier 4.

Then, to the carrier 4 which is a mixture thus mixed by the stirring bar 102 in the beaker 101, the ATP eliminating reagent is added via a pipette 103 to eliminate ATP from the carrier 4 (S4: mixture ATP eliminating step in FIGS. 5 and 6). Note, a tool other than the pipette 103 may be used for adding the ATP eliminating reagent, as long as a predetermined amount of the ATP eliminating reagent can be added. Here, there is possibility that residual ATP is contained in the raw materials of the carrier 4. Thus, the residual ATP is eliminated in the mixture ATP eliminating step S4. In the step S4, used is the beaker 101 from which residual ATP has been already eliminated in the step S2. Therefore, the ATP only in the carrier 4 can be efficiently eliminated.

The ATP eliminating reagent includes at least one selected from a group of apyrase, alkaline phosphatase, acidic phosphatase, hexokinase, adenosine triphosphatase, and adenosine phosphate deaminase

Next, the carrier 4 thus treated in the mixture ATP eliminating step S4 is injected into the tank 104a of the tubing pump 104 (S5: tank injecting step in FIGS. 5 and 6).

Then, the carrier 4 is filled in the collection dish 3 of the carrier unit 1 in the state of opening the carrier cover 5 (S6: filling step in FIGS. 5 and 6). Herein, a filling device other than the tubing pump 104 may be used as long as such a device can fill a predetermined amount of the carrier 4 into the collection dish 3. Note, the tool sterilizing step S1 and the tool ATP eliminating step S2 have to be performed as fitted to the property of the filling device.

After the carrier 4 is covered with the carrier cover 5, the carrier unit 1 filled with the carrier 4 is cooled to a temperature at which the carrier 4 is transformed into a gel phase (S7: molding step in FIGS. 5 and 6). At that time, the carrier unit 1 filled with the carrier 4 is put on the belt conveyer 106 running in an area kept at a cooling temperature thereby to be conveyed to the next operation area under the sterile condition. Here, the cooling time can be kept constant by controlling the speed of the belt conveyer 106. Note that in the present Example, the belt conveyer 106 is used in the molding step S7. However, the present invention is not limited to this configuration, as long as the cooling time can be kept constant via timer management, ect.

Next, the carrier unit 1 and the filter unit 11 thus treated in the molding step are packed (S8: packing step in FIGS. 5 and 6).

The carrier unit 1 and the filter unit 11 thus treated in the packing step S8 are subjected to gamma ray sterilization (S9: sterilizing step of target collecting instrument in FIGS. 5 and 6). Note, in the present Example, gamma ray sterilization is used as a sterilization method. However, the present invention is not limited to this method, but electron beam sterilization and EGO sterilization may be used as fitted to the property of a sterilization target.

Finally, the target collecting instrument thus treated in the sterilizing step of target collecting instrument S9 is stored up to the delivery (S10: storing step of target collecting instrument in FIGS. 5 and 6). Note, the carrier unit 1 holding the carrier 4 of performing a sol-gel transformation depending on a temperature is stored in a refrigerator.

As described above, in the present Example 1, the residual ATP is thoroughly eliminated by not only eliminating the ATP from the tools to be used in the production steps but also eliminating the ATP from the carrier 4 (i.e., raw materials). This thorough treatment can extremely reduce the possibility that ATP is contained in the carrier 4.

Here, the carrier unit 1 and the filter unit 11 thus produced are delivered without including any ATP since no ATP is remained through the production steps. Use of such a carrier unit 1 and filter unit 11 enables an extremely small amount of ATP to be measured with high accuracy.

Meanwhile, when a method includes all of the steps as described above, it is a method for producing an instrument of collecting detection target (or called target collecting instrument), including a carrier unit holding a carrier collecting a detection target.

The method includes a tool sterilizing step of sterilizing tools to be used for producing the target collecting instrument, a tool ATP eliminating step of eliminating ATP from the tools, a raw material mixing step of mixing raw materials by using the tools, a mixture ATP eliminating step of eliminating ATP from the mixture thus treated in the mixing step, a tank injecting step of injecting the mixture thus treated in the mixture ATP eliminating step into a tank of a filling device.

The method further includes a filling step of filling the mixture into the carrier unit, a molding step of molding the mixture filled in the carrier unit, a packing step of packing the target collecting instrument thus made after the molding, a target collecting instrument sterilizing step of sterilizing the target collecting instrument thus treated in the packing step, and a storing step of storing the target collecting instrument thus treated in the previous sterilizing step up to the delivery.

### (Example 2)

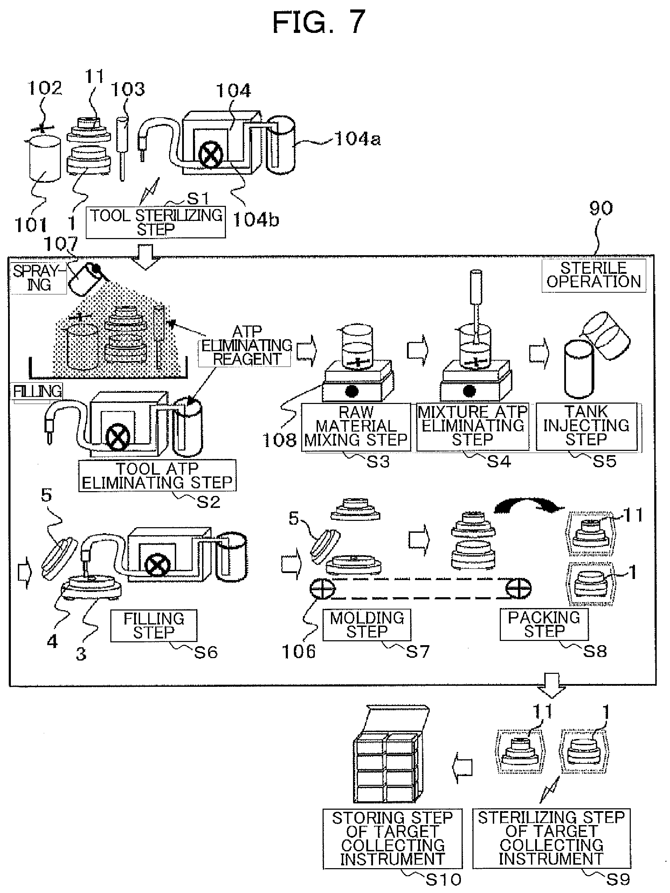

Referring to FIGS. 5 and 7, Example 2 of the present invention will be described in detail. First of all, as shown in FIG. 7, tools to be used in the production steps are prepared. The tools to be used in the production steps include a beaker 101, a stirring bar 102 for mixing raw materials, a pipette 103 for adding a reagent, a carrier unit 1 excluding a carrier 4 included in the instrument of collecting detection target (or called target collecting instrument), a filter unit 11, a tubing pump 104 for filling the carrier 4, and a tank 104a and a tube 104b both of which are components of the tubing pump 104.

At the first step, each of the tools to be used in the production steps is sterilized. The sterilization is performed by EOG sterilization (S1: tool sterilizing step in FIGS 5 and 7). In the sterilizing step S1, microorganisms including ATP are sterilized, thereby to eliminate a source of ATP generation.

Next, each of the above tools is brought to a clean bench 90 in a clean room serving as a sterile operation area with keeping the sterile condition made by the sterilizing step as described above.

Then, a liquid ATP eliminating reagent at a predetermined concentration (e.g., attached to Lucifell HS Set (60315)™ / Kikkoman) is filled in a spray container 107. Then, the liquid ATP eliminating reagent is sprayed onto a surface of each tool by the spray container 107. Note that when it is difficult to spray the liquid reagent onto contact surfaces of the tools to be in contact with raw materials of the carrier 4, the liquid ATP eliminating reagent is filled to cover the contact surfaces of the target tools (i.e., tubing pump 104, tank 104a and tube 104b).

It is possible to eliminate ATP from the contact surfaces of the tools to be in contact with the raw materials of the carrier 4 by spraying the ATP eliminating reagent onto a surface of each tool and filling the ATP eliminating reagent to cover the contact surfaces of the tools (S2: tool ATP eliminating step in FIG. 7). In the tool ATP eliminating step S2, residual ATP independently of microorganisms may be eliminated.

In Example 2, the ATP eliminating operation is conducted by the spray container 107. Use of the spray container 107 facilitates the ATP eliminating operation in the step S2 to be more easily performed than that in Example 1.

Then, the raw materials of the carrier 4 and the stirring bar 102 are put in the beaker 101. The raw materials of the carrier 4 are stirred and mixed by the stirrer 108 (S3: raw material mixing step in FIGS. 5 and 7). The carrier 4 is in a gel state at the time of collection, while it is transformed into a sol state at the time of measurement (i.e., performs a sol-gel transformation). When gelatin, glycerol, distilled water are used as the raw materials, a carrier that temperature-dependently performs a sol-gel transformation may be prepared.

Next, to the carrier 4 which is a mixture mixed by the stirring bar 102 in the beaker 101, the ATP eliminating reagent is added by the pipette 103, thereby to eliminate ATP from the mixture (S4: mixture ATP eliminating step in FIGS. 5 and 7). Since there is possibility that ATP remains in the raw materials of the carrier 4, the remaining ATP can be eliminated in the mixture ATP eliminating step S4.

The carrier 4 thus treated in the ATP eliminating step S4 is injected into the tank 104a of the tubing pump 104 (S5: tank injecting step in FIGS. 5 and 7).

The carrier 4 is filled with the collection dish 3 of the carrier unit 1 (S6: filling step in FIGS. 5 and 7).

After the carrier unit 1 is filled with the carrier 4, the carrier 4 is covered by the carrier cover 5, and molded by being cooled until the carrier 4 is transformed in a gel state. At that time, the carrier unit 1 filled with the carrier 4 is put on the belt conveyer 106 running in a space kept at a cooling temperature, and then conveyed under the sterile condition to the next operation space. Here, it is possible to keep the cooling time constant by controlling the speed of the belt conveyer 106.

Next, the carrier unit 1 and the filter unit 11 thus treated in the molding step S7 are packed, respectively (S8: packing step in FIGS. 5 and 7).

The carrier unit 1 and the filter unit 11 thus packed in the packing step S8 are subjected to gamma ray sterilization (S9: sterilizing step of target collecting instrument in FIGS. 5 and 7).

Finally, the target collecting instrument thus treated in the sterilizing step of target collecting instrument S9 is stored up to the delivery thereof (S10 : storing step of target collecting instrument in FIGS. 5 and 7). Note that the carrier unit 1 holding the carrier 4 which temperature-dependently performs a sol-gel transformation is stored in a refrigerator.

As described hereinbefore, similarly to Example 1, in the present Example 2, the residual ATP is thoroughly eliminated by not only eliminating the ATP from the tools to be used in the production steps but also eliminating the ATP from the carrier 4 (i.e., raw materials). This thorough ATP eliminating procedure enables the carrier unit 1 and the filter unit 11 thus produced to be delivered including no remaining ATP. Use of such a carrier unit 1 and filter unit 11 allows an extremely small amount of ATP to be measured with high accuracy.

Note that the ATP eliminating reagent used in the present Example 2 is the same as in Example 1. Further, the alternatives and modifications described in Example 1 are applicable to Example 2. Accordingly, the method for producing the instrument of collecting detection target (or called the target collecting instrument) in Example 2 including each of the above described steps is the same as the method for producing the target collecting instrument including each of the steps in Example 1.

### Reference Signs List

- S1: Tool Sterilizing Step
- S2: Tool ATP Eliminating Step
- S3: Raw Material Mixing Step
- S4: Mixture ATP Eliminating Step
- S5: Tank Injecting Step
- S6: Filling Step
- S7: Molding Step
- S8: Packing Step
- S9: Sterilizing Step of Target Collecting Instrument
- S10: Storing Step of Target Collecting Instrument
- 1/11: Instrument of Collecting Detection Target (or Target Collecting Instrument)
- 1: Carrier Unit
- 2: Lid
- 3: Collection Dish
- 4: Carrier
- 5: Carrier Cover
- 11: Filter Unit
- 12: Housing
- 13: Filter
- 13a: Hydrophilic Filter
- 13b: Hydrophobic Filter
- 14: Filter Fixing Ring
- 21: Connector
- 40: Air Sampler
- 41: Air Sampler Body
- 42: Air Sampler Lid
- 43: Connector Receiver
- 50: Microorganism Counter
- 90: Clean Bench
- 101: Beaker
- 102: Stirring Bar
- 103: Pipette
- 104: Tubing Pump
- 104a: Tank
- 104b: Tube
- 105: Immersion Container
- 106: Belt Conveyer
- 107: Spray Container
- 108: Stirrer

## Claims

1. A method for producing an instrument of collecting detection target, the instrument including a carrier unit holding a carrier collecting a detection target,
the method comprising:
a tool ATP eliminating step of eliminating ATP of tools to be used in production steps; and
a carrier ATP eliminating step of eliminating ATP from the carrier prepared by mixing raw materials via using the tools from which ATP is thus eliminated in the tool ATP eliminating step.

2. The method for producing the instrument of collecting detection target described in claim 1, further comprising a filling step of filling the carrier from which ATP is thus eliminated in the carrier ATP eliminating step into the carrier unit.

3. The method for producing the instrument of collecting detection target described in claim 2, further comprising a molding step of molding the carrier thus filled in the carrier unit in the filling step.

4. The method for producing the instrument of collecting detection target described in any one of claims 1 - 3, wherein the tool ATP eliminating step is performed by immersing the tools in a liquid ATP eliminating reagent to eliminate ATP from the tools.

5. The method for producing the instrument of collecting detection target described in any one of claims 1 - 3, wherein the tool ATP eliminating step is performed by spraying a misty ATP eliminating reagent onto the tools to eliminate ATP from the tools.

6. The method for producing the instrument of collecting detection target described in claim 4 or claim 5, wherein the ATP eliminating reagent includes at least one selected from a group of apyrase, alkaline phosphatase, acidic phosphatase, hexokinase, adenosine triphosphatase, and adenosine phosphate deaminase.

7. The method for producing the instrument of collecting detection target described in any one of claims 1 - 3, wherein the carrier ATP eliminating step is performed by adding an ATP eliminating reagent to the carrier thus prepared by mixing raw materials to eliminate ATP from the carrier.

8. The method for producing the instrument of collecting detection target described in claim 7, wherein the ATP eliminating reagent includes at least one selected from a group of apyrase, alkaline phosphatase, acidic phosphatase, hexokinase, adenosine triphosphatase, and adenosine phosphate deaminase.

9. The method for producing the instrument of collecting detection target described in claim 3, further comprising:
a tool sterilizing step of sterilizing the tools to be used in the production steps, the tool sterilizing step being performed prior to the tool ATP eliminating step;
a packing step of packing the instrument of collecting detection target thus treated in the molding step; and
a sterilizing step of sterilizing the instrument of collecting detection target thus packed in the packing step.

10. The method for producing the instrument of collecting detection target described in claim 9, wherein the steps from the tool ATP eliminating step to the packing step are performed in a sterile operation area.
